# EUROPEAN PATENT SPECIFICATION

(11) **EP 2 739 266 B1**
(45) Date of publication and mention of the grant of the patent: **27.07.2016**
(21) Application number: 12819309.1
(22) Date of filing: 03.08.2012
(51) Int. Cl.: A61K 9/00, A61K 31/137, A61K 31/192, A61K 31/4174, A61K 47/10, A61K 9/08

(54) **STABLE ANTI-INFLAMMATORY SOLUTIONS FOR INJECTION**
STABILE ENTZÜNDUNGSHEMMENDE LÖSUNGEN ZUR INJEKTION
SOLUTIONS INJECTABLES ANTI-INFLAMMATOIRES STABLES

(30) Priority: 04.08.2011 US 201161515234 P
(43) Date of publication of application: 11.06.2014
(73) Proprietor: Omeros Corporation, Seattle, WA 98119 (US)
(72) Inventor: DEMOPULOS, Gregory, A., Mercer Island, WA 98040 (US); GOMBOTZ, Wayne, R., Kenmore, WA 98028 (US); SHEN, Hui-rong, Bothell, WA 98021 (US)
(74) Representative: Cole, William Gwyn
(86) International application number: PCT/US2012/049463
(87) International publication number: WO 2013/020021

(56) References cited:
- US-A1- 2007 207 222
- US-A1- 2011 033 540
- US-A1- 2012 208 858
- US-B2- 7 091 181
- US-B2- 7 973 068
- FANTON G S ET AL: "Novel Drug Product to Improve Joint Motion and Function and Reduce Pain After Arthroscopic Anterior Cruciate Ligament Reconstruction", ARTHROSCOPY, RAVEN PRESS, NEW YORK, NY, US, vol. 24, no. 6, 1 June 2008 (2008-06-01), pages 625-636, XP022675983, ISSN: 0749-8063, DOI: 10.1016/J.ARTHRO.2008.02.003 [retrieved on 2008-05-28]
- WILLIAM E GARRETT ET AL: "Novel Drug OMS103HP Reduces Pain and Improves Joint Motion and Function for 90 Days After Arthroscopic Meniscectomy", ARTHROSCOPY, RAVEN PRESS, NEW YORK, NY, US, vol. 27, no. 8, 24 February 2011 (2011-02-24), pages 1060-1070, XP028249225, ISSN: 0749-8063, DOI: 10.1016/J.ARTHRO.2011.02.038 [retrieved on 2011-03-11]

## Description

### I. Field of the Invention

The present invention relates to stable liquid pharmaceutical formulations of ketoprofen, amitriptyline, and oxymetazoline for injection.

### II. Background of the Invention

Pharmaceutical agents are sometimes administered directly to a localized site of inflammation or a site in which trauma will likely result in inflammation. Administration of pharmaceutical agents can occur via injection or other means. For example, pharmaceutical agents can be administered during arthroscopic procedures. Arthroscopy is a surgical procedure in which a camera, attached to a remote light source and video monitor, is inserted into an anatomic joint (e.g., knee, shoulder, etc.) through a small portal incision in the overlying skin and joint capsule. Throughout each arthroscopy, physiologic irrigation fluid (e.g., normal saline, lactated Ringer's or glycine) is flushed continuously through the joint, distending the joint capsule and removing operative debris, thereby providing clearer intra-articular visualization.

Irrigation is also used in other procedures, such as cardiovascular and general vascular diagnostic and therapeutic procedures, urologic procedures and the treatment of bums and any operative wounds. In each case, a physiologic fluid is used to irrigate a wound or body cavity or passage. Conventional physiologic irrigation fluids do not provide analgesic, anti-inflammatory effects.

Conventional physiologic irrigation fluids are not used to administer therapeutic agents to a patient. However, dilute irrigation solutions have been used to deliver therapeutic agents directly to a surgical site during a surgical procedure. See, e.g., International PCT Patent Pubs. WO96/19,233 A2, WO97/21,445 A1 and WO00/23,066 A2, US Pat. Pubs. 20030096807 A1, 20030087962A1 and 200320090253795 A1, and US Patent Nos. 5,860,950, 6,645,168 and 7,973,068, each to Demopulos et al. A combination of three drugs, ketoprofen, amitriptyline, and oxymetazoline, in a dilute solution in a physiologic carrier, with each pharmacologic agent being present in the irrigation solution at a concentration of no more than 100,000 nanomolar, is disclosed in US Patent 7,973,068.

This drug combination (ketoprofen, amitriptyline, and oxymetazoline) has been shown to improve postoperative function when administered directly to a knee joint in a dilute irrigation solution perfused through the joint during arthroscopic surgery. A solution including ketoprofen, amitriptyline, and oxymetazoline at concentrations of 4.55 µg/mL, 1.50 µg/mL, and 1.42 µg/mL, respectively, in an irrigation carrier was perfused through the joint during arthroscopic surgery. Patients receiving the three drug combination during arthroscopic anterior cruciate ligament reconstruction surgery demonstrated significant postoperative improvements in knee function, range of motion and pain reduction as compared to patients receiving non-medicated irrigation vehicle alone. Fanton et al., Arthroscopy 24:625-636 (2008). Patients receiving the same three drug combination in a dilute irrigation solution perfused through the knee joint during partial meniscectomy surgery exhibited statistically significant improvements relative to irrigation vehicle as measured by patient-reported functional scores using the Knee Injury and Osteoarthritis Outcome Score (KOOS), passive knee flexion and pain assessed by visual analog scale scores. The patient-reported outcomes measuring symptoms, pain, activities of daily living, sport and recreational activities and quality of life associated with the operated knee showed a sustained benefit through postoperative Day 90. Garrett et al., Arthroscopy 27:1060-1070 (2011).The solution administered in these studies was formulated as a lyophilized product. The lyophilized drug combination was then reconstituted in lactated Ringer's solution and injected into a bag of irrigation fluid prior to administration to patients in this very dilute form throughout the arthroscopic surgery. At the end of the surgical procedure, 30 mL of this irrigation solution were administered into the knee by intra-articular injection through a previously closed portal site, delivering an additional small amount of ketoprofen (137 µg), amitriptyline (45 µg) and oxymetazoline (43 µg) to provide continued inhibition of the inflammatory response at the surgical site.

Therapeutic agents can also be delivered directly to a desired local anatomic site by injection. For example corticosteroids have been injected into soft tissue or joints to successfully treat a variety of inflammatory conditions, e.g., synovitis, arthritis, bursitis, tendonitis, carpal tunnel syndrome, fasciitis, gangliotic cysts and neuromas. Salinas and Rosenburg, emedicine.medscape.com/article/325370-overview, (2009), accessed September 16, 2010. However, local injection of corticosteroids into joints has been associated with cartilage damage, and accordingly many practitioners limit the number of such injections. *See, e.g.,* Shapiro, The Effect of Local Corticosteroid or Ketorolac Exposure on Histologic and Biomechanical Properties of Rabbit Tendon and Cartilage, Hand, 2(4): 165-172 (2007), concluding that the use of an injectable nonsteroidal anti-inflammatory agent may pose less threat to local tissues after intra-articular and peri-tendinous administration.

Surgical irrigation and direct injection into a joint are typically performed with liquid formulations of pharmaceutical agents. The three-drug combination administered by Fanton et al. and Garrett et al., i.e., ketoprofen, amitriptyline, and oxymetazoline, was solubilized in irrigation fluid from a lyophilized state. While these three drugs have demonstrated therapeutic benefits, they also have characteristics that make design of a stable and cost-effective drug formulation challenging, particularly a stable liquid formulation. For example, ketoprofen is minimally soluble in aqueous solution and is more soluble above pH 6.0. During manufacturing, ketoprofen is difficult to wet and dissolve into aqueous media, even in the presence of arginine and lysine to form ketoprofen salt. Amitriptyline HCl is generally chemically unstable in aqueous solution and is less stable when stored above pH 6.0. In addition, amitriptyline also exhibits physical instability by forming micelle particles, thus suffering a decrease in concentration under certain conditions (see attached EP patent # 0 431 663 B1). Most drugs formulated in solutions are less stable than drugs formulated in a lyophilized or dry state, particularly when the solution is an aqueous solution in which the drugs are more susceptible to chemical degradation reactions such as hydrolysis and oxidation. The present invention solves these and other problems.

### III. Summary of the Invention

The present invention provides liquid pharmaceutical formulations of ketoprofen, amitriptyline, and oxymetazoline that are stable for at least six months when stored at a range of 2°C to 30°C. The ketoprofen, amitriptyline, and oxymetazoline are also soluble in these formulations. The formulations include 1-99% (v/v) polyol, and 10-500 mM sodium citrate (Na citrate) at a pH of 4.5-7.0. In one embodiment, the pharmaceutical formulations contain 550 µM to 1 M ketoprofen, 55 µM to 1 M amitriptyline, and 350 µM to 1 M oxymetazoline in liquid solution. In one embodiment of the invention, the liquid formulation is an aqueous solution.

In one embodiment, the pharmaceutical formulations contain 10-70% (v/v) polyol. In another embodiment, the pharmaceutical formulations contain 15-25% (v/v) polyol. In a further embodiment, the pharmaceutical formulation contains 20% (v/v) polyol.

A preferred polyol for use in the present invention is polyethylene glycol (PEG), e.g., PEG 400. In one embodiment, the pharmaceutical formulations contain 10-70% (v/v) PEG, e.g., 10-70% (v/v) PEG 400. In another embodiment, the pharmaceutical formulations contain 15-25% (v/v) PEG, e.g., 15-25% (v/v) PEG 400. In a further embodiment, the pharmaceutical formulation contains 20% (v/v) PEG, e.g., 20% (v/v) PEG 400.

In one embodiment of the invention, the formulation is an aqueous solution including water in addition to a poylol as the liquid medium. In one embodiment, the pharmaceutical formulations contain 30-90% (v/v) water. In another embodiment, the pharmaceutical formulations contain 75-85% (v/v) water. In a further embodiment, the pharmaceutical formulation contains 80% (v/v) water.

In one embodiment of the invention, the pH of the pharmaceutical formulation is between 5.0 and 6.2. In another embodiment of the invention, the pH of the pharmaceutical formulation is between 5.2 and 5.8. In a further embodiment of the invention, the pH of the pharmaceutical formulation is between 5.4 and 5.6. In a preferred embodiment of the invention the pH of the pharmaceutical formulation is about 5.5.

The liquid pharmaceutical formulations of the invention are very stable. For example, in one embodiment, the ketoprofen, amitriptyline, and oxymetazoline are stable for at least twelve months when stored at a range of 2°C to 30°C. In another embodiment, the ketoprofen, amitriptyline, and oxymetazoline are stable for at least eighteen months when stored at a range of 2°C to 30°C. In another embodiment, the ketoprofen, amitriptyline, and oxymetazoline are stable for at least twenty-four months when stored at a range of 2°C to 30°C. In further embodiments, the ketoprofen, amitriptyline, and oxymetazoline are stable for at least thirty months or for at least thirty-six months, or for at least forty-eight months when stored at a range of 2°C to 30°C.

In one embodiment, the pharmaceutical formulations include 0.002-1.0% (w/v) sodium ethylenediamine tetraacetic acid (Na₂ EDTA). In another embodiment, the pharmaceutical formulations include 0.01-0.5% (w/v) Na₂ EDTA. In a further embodiment, the pharmaceutical formulations include 0.05% (w/v) Na₂ EDTA.

In one embodiment, the pharmaceutical formulations include about 50 mM sodium citrate (Na Citrate).

In one aspect, the pharmaceutical formulations are exposed to nitrogen gas (N₂) before storage to enhance stability. In a further aspect, the pharmaceutical formulations are stored in the dark, also to enhance stability.

In one aspect, the pharmaceutical formulations are prepared in a manner that provides for dilution into an irrigation solution for use during a surgical procedure. In another aspect, the pharmaceutical formulation is prepared for direct injection into a localized site of inflammation, at a concentration that may be the same as that used after dilution in an irrigation vehicle for irrigation or that may be more concentrated, such as the concentration of the pharmaceutical formulation prior to dilution. The site of the inflamed tissue to be treated by local injection in accordance with the present invention may be an articular joint, a peritendinous or periligamentous area, fascia or other connective tissue, muscle, or other tissue. In one embodiment, the localized site of inflammation is an acutely injured tendon or peritendinous area, ligament or periligamentous area, connective tissue, muscle or joint of an extremity, such as an ankle, knee, hip, wrist, elbow, shoulder, or temporomandibular joint, or another joint within the body. In another embodiment, the localized site of inflammation is a site inflamed due to a subacute or chronic condition, such as, for example, osteoarthritis, rheumatoid arthritis, arthrofibrosis, frozen joints, flexor tenosynovitis, plantar fasciitis, bursitis, tendonitis, carpal tunnel syndrome, gangliotic cysts, chronic back pain, and repetitive stress or overuse injuries.

An aspect of the invention provides a method of preventing or treating inflammation at an anatomic site that is inflamed or at risk of inflammation, by injecting into the site a composition comprising the anti-inflammatory agents ketoprofen, amitriptyline and oxymetazoline in a physiologic carrier, in the absence of a surgical procedure such that the composition is retained at the site for a period of time sufficient, and in which wherein the agents are injected in an amount sufficient, to inhibit inflammation at the site.

In one aspect the pharmaceutical formulations are part of a kit that includes instructions for use. The pharmaceutical formulation can be packaged in a single dose syringe for direct injection into a localized site of inflammation. Alternatively, the pharmaceutical formulation can be packaged in a vial, cartridge or ampoule.

In one aspect, the present invention provides liquid pharmaceutical formulations of ketoprofen, amitriptyline, and oxymetazoline that are stable for at least six months when stored at 2-30°C. The formulations include 0.1-1.0 g/L ketoprofen, 0.05-0.5 g/L amitriptyline, 0.05-0.5 g/L oxymetazoline, 1-99% (v/v) PEG 400, about 0.002-1.0% (w/v) Na₂EDTA, and 10-500 mM Na citrate, pH 5.0-6.2. The ketoprofen, amitriptyline, and oxymetazoline are soluble in this stable liquid formulation.

In another aspect, the present invention provides liquid pharmaceutical formulations of ketoprofen, amitriptyline, and oxymetazoline that are stable for at least six months when stored at a temperature of 2-30°C. The formulations include about 0.687 g/L ketoprofen, about 0.227 g/L amitriptyline, about 0.215 g/L oxymetazoline, 20% (v/v) PEG 400, about 0.05% (w/v) Na₂EDTA, and about 50 mM Na citrate, pH 5.0-6.2. The ketoprofen, amitriptyline, and oxymetazoline are soluble in the liquid formulation.

### IV. Brief Description of the Drawings

The present invention will now be described in greater detail, by way of example, with reference to the accompanying drawings in which:

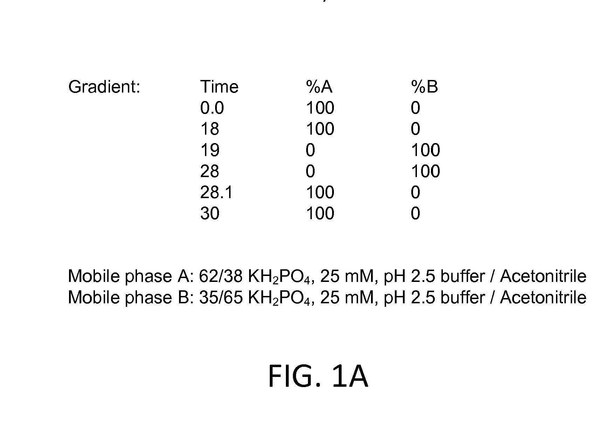

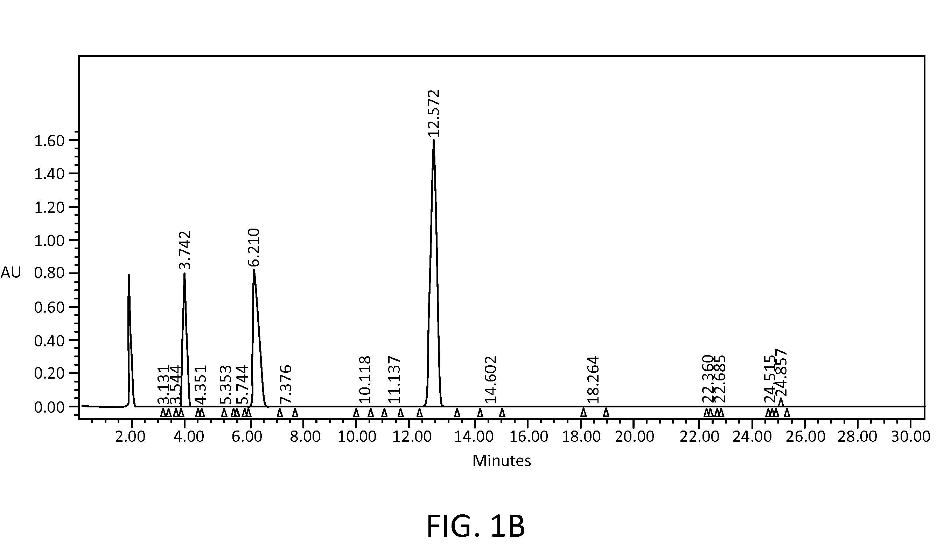

FIGURE 1A and 1B provide conditions and results of gradient high performance liquid chromatography (HPLC) used to quantify ketoprofen, amitriptyline HCl, and oxymetazoline HCl and their related substances. Figure 1A provides the gradient and elution buffers used. Figure 1B shows a representative chromatogram and peaks.

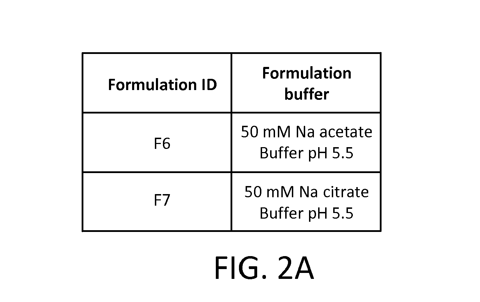

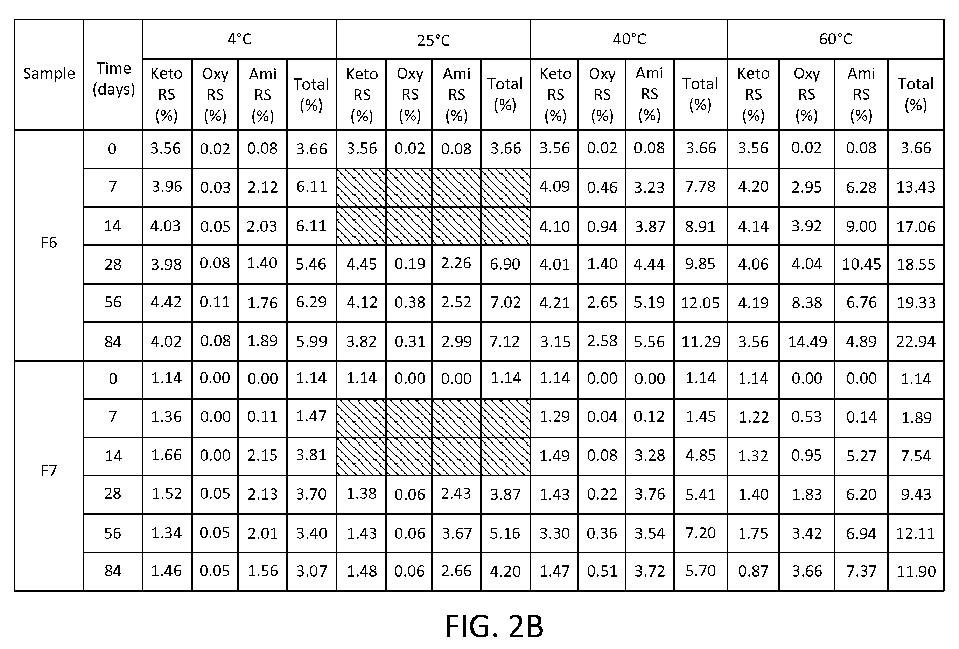

FIGURE 2A and 2B provide the results of storing ketoprofen, amitriptyline HCl, and oxymetazoline HCl in liquid pharmaceutical formulations containing different buffers. The formulations and tested buffers are provided in Figure 2A. Figure 2B provides the percentage of related substances for each drug component found after storage for eighty-four days at 4°C, 25°C, 40°C, or 60°C.

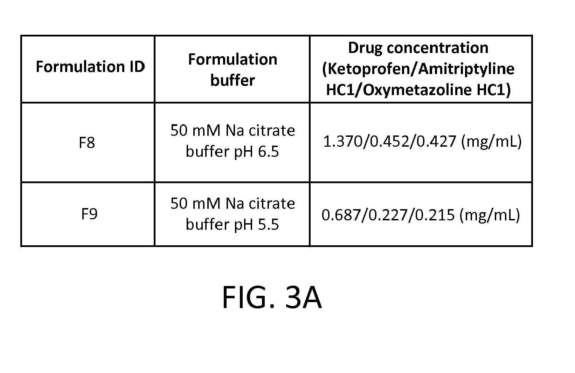

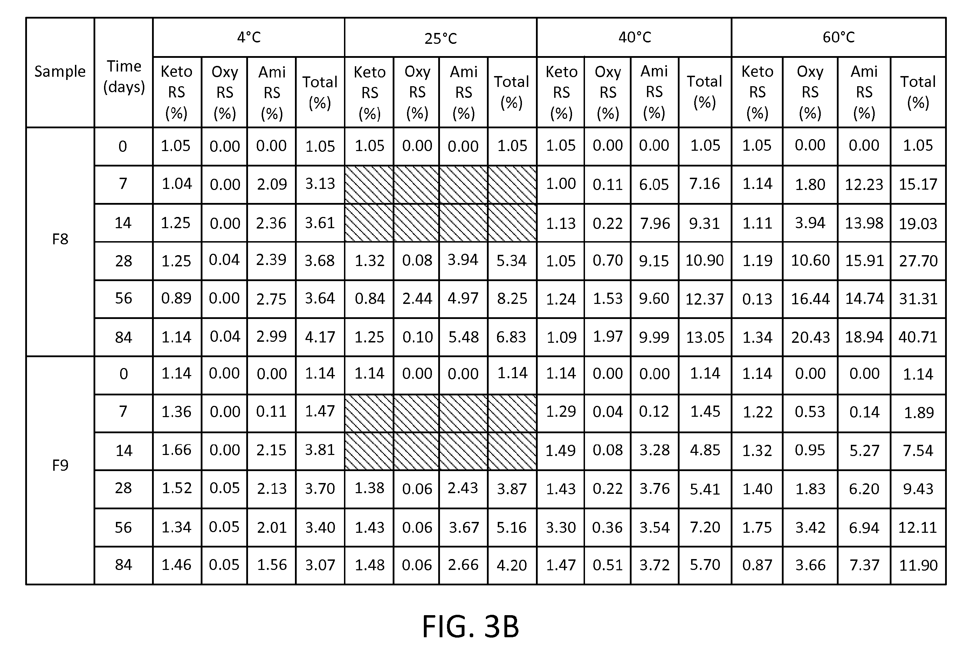

FIGURE 3A and 3B provide the results of storing ketoprofen, amitriptyline HCl, and oxymetazoline HCl in liquid pharmaceutical formulations at different pH values. The formulations and tested pH values are provided in Figure 3A. Figure 3B provides the percentage of related substances for each drug component found after storage for eighty-four days at 4°C, 25°C, 40°C, or 60°C.

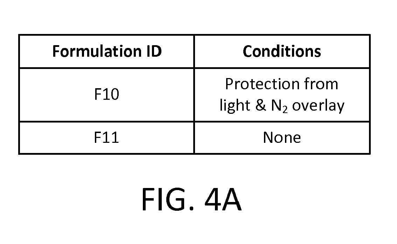

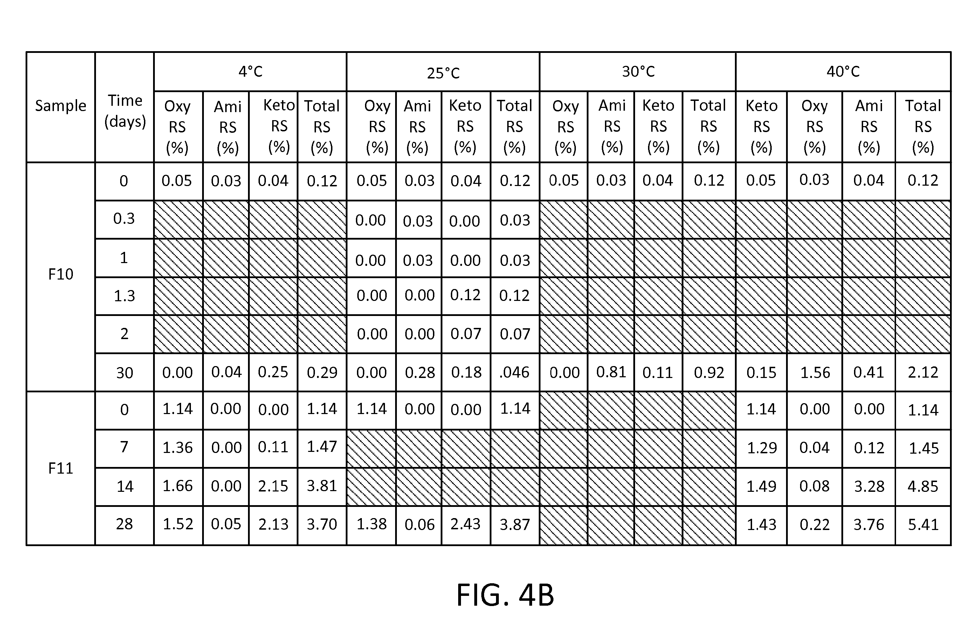

FIGURE 4A and 4B provide the results of storing ketoprofen, amitriptyline HCl, and oxymetazoline HCl in liquid pharmaceutical formulations in the presence or absence of light and with or without a N₂ overlay. The formulations and fill/storage conditions are provided in Figure 4A. Figure 4B provides the percentage of related substances for each drug component found after storage for twenty-eight days at 4°C, 25°C, 30°C, or 40°C.

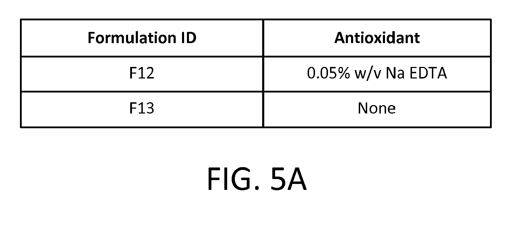

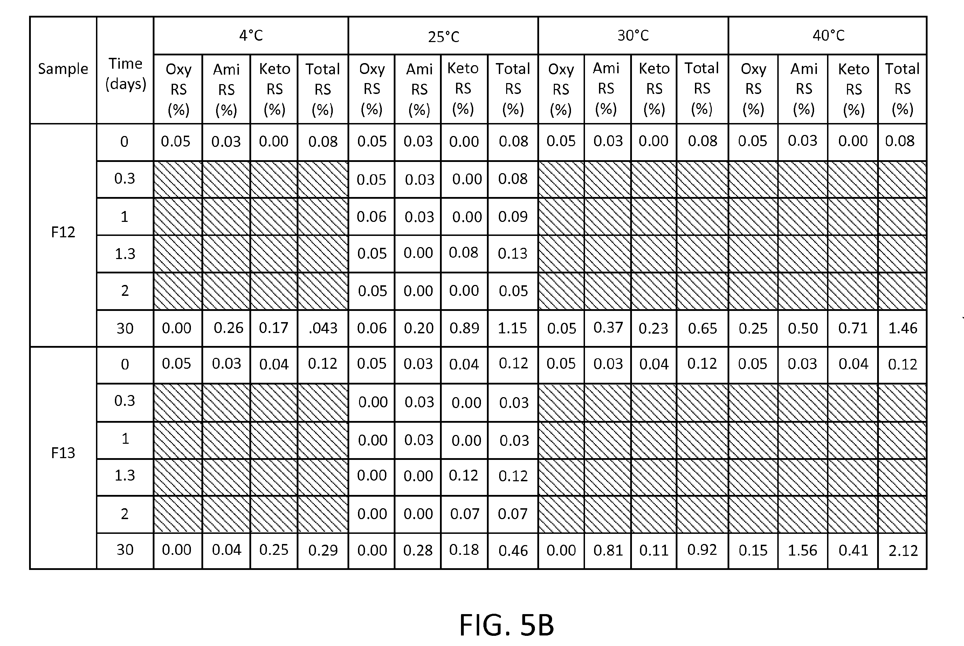

FIGURE 5A and 5B provide the results of storing ketoprofen, amitriptyline HCl, and oxymetazoline HCl in liquid pharmaceutical formulations in the presence or absence of Na₂ EDTA. The formulations and Na₂ EDTA concentrations are provided in Figure 5A. Figure 4B provides the percentages of related substances for each drug component found after storage for thirty days at 4°C, 25°C, 30°C, or 40°C.

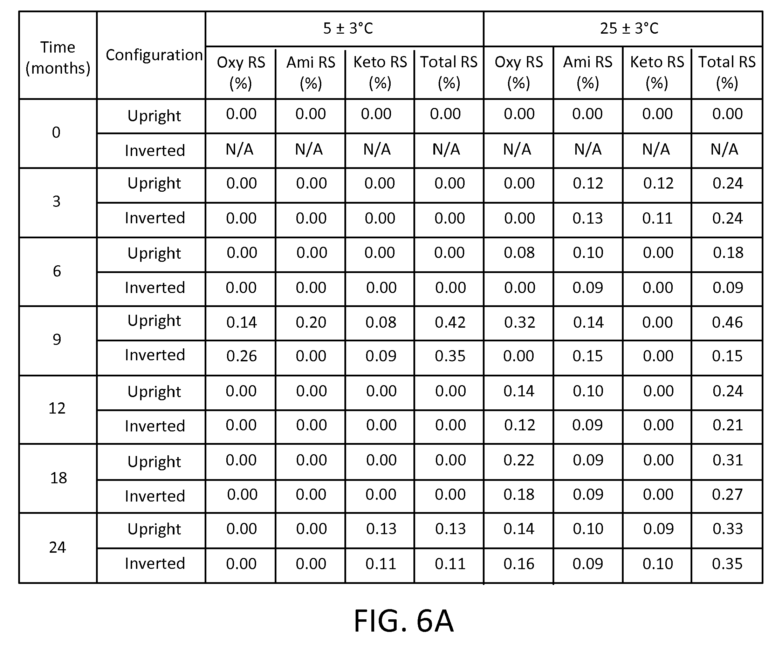

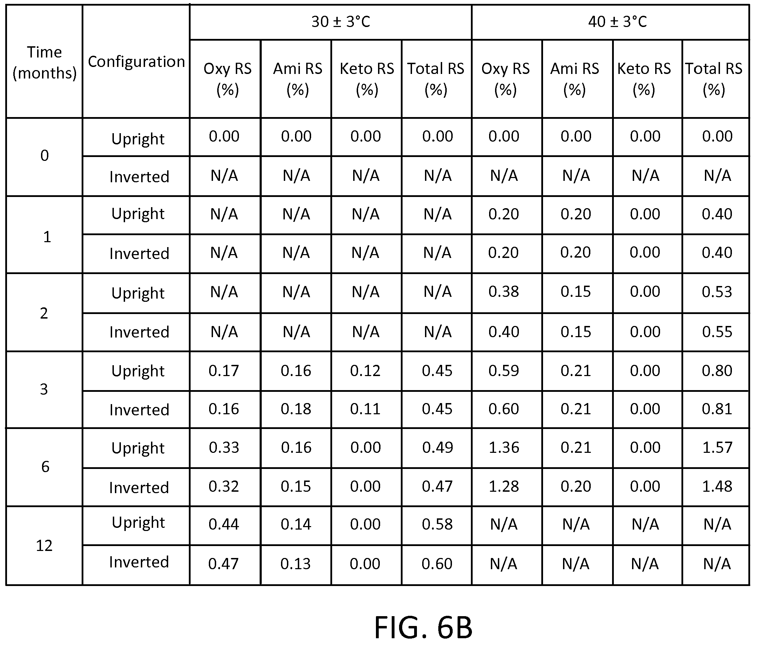

FIGURE 6A and 6B provide the results of storing a liquid pharmaceutical formulation comprising ketoprofen, amitriptyline HCl, and oxymetazoline HCl. Results shown are related substances for one representative lot of the pharmaceutical formulation of the present invention. The components of the pharmaceutical formulation are about 50mM Na citrate, pH 5.5; 20% v/v PEG 400, 0.05% w/v Na₂ EDTA, Ketoprofen (0.687 mg/mL), Amitriptyline HCl (0.227 mg/mL), and Oxymetazoline HCl, and (0.227 mg/mL). Aliquots in vials were stored for at least twenty-four months at 5°C and 25°C and for up to twelve months at 30°C and 40°C. Vials were stored in upright or inverted position.

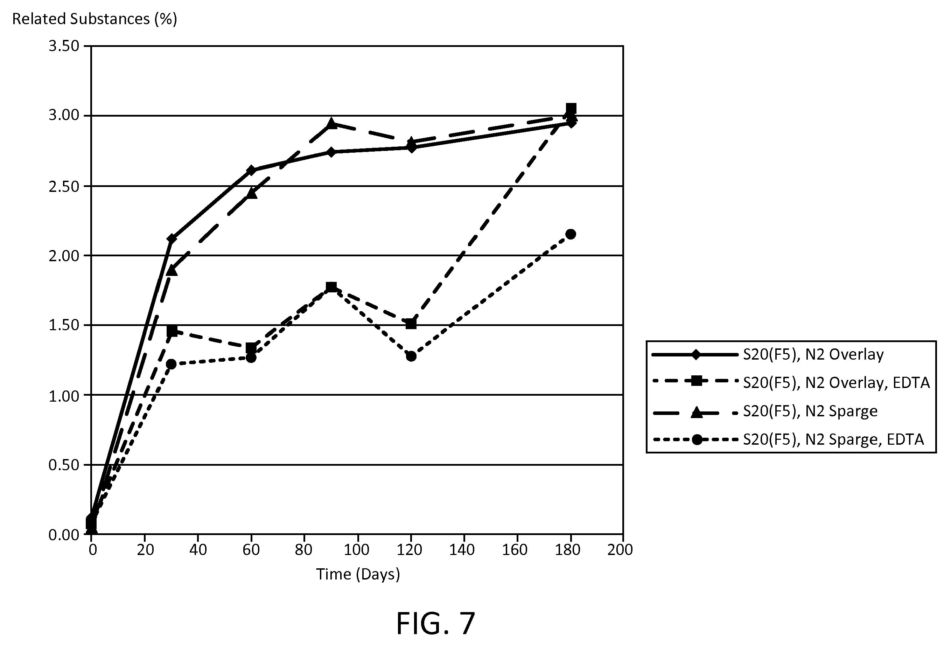

FIGURE 7 provides analysis of total related substances in liquid pharmaceutical formulations with or without Na₂ EDTA. Samples were either overlaid or sparged with N₂.

### V. Detailed Description

### Introduction

The present disclosure provides for the first time, liquid pharmaceutical formulations of ketoprofen, amitriptyline, and oxymetazoline (the three active pharmaceutical ingredients, or "APIs") that are stable for at least six months when stored between 2°C and 30°C. In some embodiments, the disclosed liquid pharmaceutical formulations are surprisingly stable for long time periods, *e.g.,* one, two, three or even four years at 25°C, when compared with a significantly less stable lyophilized drug product containing the same three active ingredients (approximately 5.9% of total degradants when stored at 25°C for 18 months). The instability of the three APIs that is addressed by the present invention is due to their susceptibility to various degradation pathways such as oxidation, acid hydrolysis, base hydrolysis, and photolysis. The prolonged stability of the drugs is unexpected given the susceptibility of most drugs to undergo chemical degradation in solutions over time, particularly aqueous solutions. The liquid pharmaceutical formulations disclosed herein are about seventeen times more stable than a lyophilized combination of the same drugs stored at similar temperatures.

### Definitions

Polyol refers to a compound containing more than one hydroxyl group. Preferred polyols include polyethylene glycol (PEG), e.g., PEG 200, PEG 300, PEG 400, PEG 540, PEG 600, PEG 900, PEG 1000, PEG 1450, PEG 1540, PEG 2000, PEG 3000, PEG 3350, PEG 4000, PEG 4600, PEG 6000, PEG 8000, PEG 20,000, and PEG 35,000; propylene glycol; ethyl alcohol; glycofurol; and diethyleneglycol mono methylether.

The stable pharmaceutical formulation is present in a liquid state. The active pharmaceutical ingredients are dissolved in a liquid medium, carrier or solvent. The liquid medium can be, e.g., a polyol, such as PEG400; water; or glycine. A stable aqueous pharmaceutical formulation comprises water as the liquid medium. Embodiments of the invention comprise a polyol and water as the liquid medium.

Stable refers to a liquid pharmaceutical formulation that, at the end of a specified storage period of time, contains less than 5% total related substances, i.e., substances that result from degradation of the active pharmaceutical ingredients ketoprofen, amitriptyline, and oxymetazoline. In one embodiment, a stable liquid formulation is stable at a temperature between 2°C and 30°C for a period of at least six months. In a preferred embodiment, a stable liquid formulation is stable at a temperature between 2°C and 30°C for a period of at least one year.

Exposed to N₂ or exposure to N₂ refers to a process by which the disclosed pharmaceutical formulations come in contact with N₂ gas, typically during the fill/finish process. The N₂ exposure can continue throughout the storage of the pharmaceutical formulation. N₂ exposure can be accomplished by a variety of methods. For example, N₂ can be bubbled through or sparged through the compounded bulk pharmaceutical formulation. Water used for the formulation can also be exposed to N₂ prior to compounding. The pharmaceutical formulation can also be overlaid with N₂, usually immediately before being stored.

The term "about" is understood to mean that there can be variation in the concentration of a component of the described formulation that can be to 5%, 10%, 15% or up to and including 20% of the given value. For example, the phrase "a formulation having about 50 mM sodium citrate" is understood to mean that the formulation can have between 40 mM to 60 mM sodium citrate.

### Pharmaceutical Agents

This invention provides stable, liquid pharmaceutical formulations of a combination of three drugs used for the inhibition of pain and inflammation during surgical, medical, diagnostic and interventional procedures and for injection into sites of localized inflammation, e.g., joints, tendons, ligaments, and other anatomic structures. The disclosed liquid formulations are stable for at least six months when stored at a temperature of between 2°C and 30°C. The three drugs are the cyclooxygenase (COX) inhibitor ketoprofen, the 5-hydroxytryptamine2A (5-HT2A) receptor antagonist amitriptyline, and oxymetazoline, which activates 5-HT1B and 5-HT1D receptors.

### Ketoprofen

Ketoprofen exhibits potent anti-inflammatory, analgesic, and antipyretic actions that are associated with the inhibition of prostaglandin synthesis and antagonism of the effects of bradykinin. Ketoprofen non-selectively inhibits the activity of COX-1 and COX-2, which results in the blockade of prostaglandin production, particularly that of PGE₂, preventing the development of hyperalgesia. Ketoprofen has an IC₅₀ value of 4 - 8 nM in a non-selective COX assay, being functionally 6 - 12 times more potent than other NSAIDs evaluated (e.g., naproxen or indomethacin). Kantor, T., Pharmacotherapy 6:93-103 (1986). Ketoprofen also has functional bradykinin antagonist activity, its effects being eight times greater than those seen with the classical NSAID, indomethacin. Julou, L., et al., Scand J Rheumatol Suppl. 0:33-44 (1976). In addition to inhibiting cyclooxygenase, ketoprofen is believed to offer the additional anti-inflammatory benefit of inhibiting lipoxygenase.

Ketoprofen is known to be minimally soluble in aqueous solutions. The predicted water solubility of ketoprofen is 0.0213 mg/ml according to the Drugbank.ca database. *See, e.g.,* Wishart et al., Nucl. Acids Res. 34:D668-672 (2006) and Wishart et al., Nucl. Acids Res. 36:D901-908 (2008). Ketoprofen has been prepared in an aqueous formulation by making an amino acid salt of the molecule. Best results were obtained using a lysine salt of ketoprofen. *See, e.g.,* US Patent No. 5,895,789. Ketoprofen has been solubilized in a 35% PEG 400 solution and in 40% ethanol. *See e.g.* U.S. Patent Applications 2003/0096807 and 2006/026339.

### Amitriptyline

Amitriptyline is a serotonin receptor antagonist that has been used clinically for numerous years as an anti-depressant, and has beneficial effects in certain chronic pain patients. Amitriptyline is included in the formulations of the present invention based on its distinct peripheral, acute, and local anti-inflammatory / analgesic effects that are mediated by its antagonist actions at multiple receptor subtypes, including adrenergic, histamine, muscarinic cholinergic, and 5-HT, that are involved in mechanisms of peripheral inflammation and pain. Prior studies have demonstrated the ability of endogenous agents, such as serotonin (5-hydroxytryptamine, sometimes referred to herein as "5-HT"), bradykinin and histamine, to produce pain and inflammation. Sicuteri, F., et al., Life Sci. 4: 309-316 (1965); Rosenthal, S.R., J. Invest. Dermat. 69:98-105 (1977); Richardson, B.P., et al., Nature 316:126-131 (1985); Whalley, E.T., et al., Naunyn-Schmiedeb Arch. Pharmacol. 36:652-57 (1987); Lang, E., et al., J. Neurophysiol. 63:887-901 (1990). Serotonin (5-HT) is thought to produce pain by stimulating serotonin₂ (5-HT₂) and/or serotonin₃ (5-HT₃) receptors on nociceptive neurons in the periphery.

Amitriptyline is known to be unstable in aqueous solutions. Attempts have been made to stabilize liquid formulations of amitriptyline by including amino acids, e.g., tryptophan or methionine, or by including a long list of buffers and preservatives, such as benzalkonium chloride, Na EDTA, sodium bisulfate, phenylmercuric acetate, cetylpyridinium chloride, thimerosal, chlorobutamol, cetyltrimethyl ammonium bromide, methylparaben, propylparaben, and butylparaben. *See, e.g.,* EP 0 431 663, GB 2,082,910, and EP 93,373.

Amitriptyline is most commonly used as an HCl salt, e.g., amitriptyline, HCl. However, the term amitriptyline, encompasses other forms of amitriptyline, including other salt forms of amitriptyline.

### Oxymetazoline

Oxymetazoline is a potent selective α2A-adrenoceptor agonist that has complex interaction with members of the α-adrenoceptor family. Hoffman and Taylor Neurotransmission. In: Hardman, Limbird, and Gilman eds. Goodman & Gilman's the Pharmacological Basis of Therapeutics. 10th Ed. 105-139 (2001) and Watling, the Sigma-RBI Handbook of Receptor classification and Signal Transduction. 4th Ed. (2001). In receptor binding assays, oxymetazoline also has potent activity at non-human 5-HT1A, 5-HT1B and 5-HT1D receptors (IC50=3-26 nM). Schoeffter and Hoyer Eur. J. Pharmcol. 196: 213-216 (1997). It is ten-fold more potent at human 5-HT1B and 5-HT1D receptors (Ki=0.3-0.4nM); and is a functional 5-HT1B/1D receptor agonist (IC50+30nM) in the human saphenous vein. Law et al., J. Med. Chem. 41:2243-2251 (1998) and Molderings et al., Nauyn Schmiedebergs Arch Pharmcol. 342:371-377 (1990).

While oxymetazoline binds potently to both α1A- and α2A-adrenoceptors (Ki =3-6nM), it is a partial agonist at both receptors and also interacts with other members of the α-adrenoceptor family. Newman-Tancredi et al., Nauyn Schmiedebergs Arch Pharmcol. 358:197-206 (1998); Bylund et al., J. Pharmacol. Exp. Ther. 281:1171-1177 (1997); Naselsky et al, J. Pharmacol. Exp. Ther. 298:219-225 (2001); Horie et al., Br. J. Pharmacol. 116:1611-1618 (1995); Ruffolo et al., J. Pharmacol. Exp. Ther. 209:429-436 (1979); Bylund et al., J. Pharmacol. Exp. Ther. 245:600-607 (1988); Ruffolo et al., J. Pharmacol. Exp. Ther. 224:559-566 (1983); and Murphy et al., J. Pharmacol. Exp. Ther. 244:571-578 (1988). Despite this, the compound is generally accepted as a selective α2A-adrenoceptor agonist. Hoffman and Taylor and Schoeffter and Hoyer, *supra***.** In addition, oxymetazoline has dose-dependent, antinociceptive/anti-inflammatory activity in several animal models of pain and inflammation. Reddy et al., J. Pharmacol. Exp. Ther. 213:525-533 (1980); Sherman et al., J. Pharmacol. Exp. Ther. 245:319-326 (1988); and Loomis et al., Brain Res. 599:73-82 (1992).

Oxymetazoline has well-established activity as a decongestant for ocular and nasal use, based on its vasoconstrictive effects. Physicians' Desk Reference, 25th Ed. (2004). The vasoconstrictive effects of oxymetazoline can also restore vascular tone occurring at sites of inflammation, including, e.g., surgical sites, acutely injured joints, chronically inflamed joints or other tissues. Najafipour, Exp. Physiol. 85:267-273 (2000). The activity of oxymetazoline at 5-HT1B/1D receptors can also inhibit the release of pro-inflammatory mediators, providing additional anti-inflammatory activity at sites of inflammation. Law et al., *supra*.

Oxymetazoline is most commonly used as an HCl salt, e.g., oxymetazoline HCl. However, the term oxymetazoline, encompasses other forms of oxymetazoline, including other salt forms of oxymetazoline.

### Formulations

The stable, liquid pharmaceutical formulations of the present invention include ketoprofen, amitriptyline, and oxymetazoline in concentrated form in a polyol medium, and may be stored and then prepared by dilution in an aqueous solution for parenteral delivery. In one preferred embodiment, the stable liquid pharmaceutical formulation is diluted in a physiologic irrigation liquid carrier to a concentration appropriate for local delivery to a surgical site. In another preferred embodiment, the stable liquid pharmaceutical formulation is either used in its concentrated stable form, or may be diluted in a physiologic liquid carrier, before administration by injection to a patient at a site of localized inflammation. In a further preferred embodiment, the stable liquid pharmaceutical formulation is prepared with a concentration of active ingredients that is appropriate for direct injection into a localized site of inflammation, either a chronically inflamed site or an acutely injured site.

The amounts of pharmaceutically active ingredients included in the formulation can be expressed in molar ratios. Typically the ratio of amitriptyline HCl and oxymetazoline HCl are 1:1. The ratios of ketoprofen to amitriptyline HCl and oxymetazoline HCl can range from 10:1:1 to 1:10:10. The ketoprofen, amitriptyline HCl and oxymetazoline HCl are suitably included in a molar ratio (ketoprofen:amitriptyline HCl:oxymetazoline HCl) of from 10:1:1 to 1:10:10, preferably from 5:1:1 to 1:5:5, more preferably from 4:1:1 to 1:1:1, and most preferably approximately (i.e., +/- 20%) 3.5:1:1. Other exemplary molar ratios are (ketoprofen:amitriptyline HCl:oxymetazoline HCl) 1:1:1, 1:2:1, 1:1:2, 2:1:1, 2:2:1, 2:1:2, 1:2:2, 1:5:1, 1:1:5, 5:1:1, 5:5:1, 5:1:5, 1:5:5, 1:10:1, 1:1:10, 10:1:1, 10:10:1, 10:1:10, 1:10:10, 1:50:1, 1:1:50, 50:1:1, 50:50:1, 50:1:50, 1:50:50, 1:100:1, 1:1:100, 100:1:1, 100:100:1, 100:1:100, and 1:100:100.

Ketoprofen is included in the stable liquid pharmaceutical formulation at concentrations between 550 µM and 1 M, preferably between 800 µM and 100 mM, more preferably between 1 mM and 10 mM, and most preferably between 1 mM and 5 mM. Amitriptyline is included in the stable liquid pharmaceutical formulation at concentrations between 55 µM and 1 M, preferably between 100 µM and 100 mM, more preferably between 200 µM and 10 mM, and most preferably between 300 µM and 1.5 mM. Oxymetazoline is included in the stable liquid pharmaceutical formulation at concentrations between 350 µM and 1 M, preferably between 350 µM and 100 mM, more preferably between 350 µM and 10 mM, and most preferably between 350 µM and 1.5 mM. In one preferred embodiment, the stable liquid pharmaceutical formulation comprises about 2.7 mM ketoprofen, about 720 µM amitriptyline, and about 720 µM oxymetazoline. In this embodiment, the molar ratio of ketoprofen:amitriptyline:oxymetazoline is about 3.5:1:1.

In accordance with another aspect of the invention, anti-inflammatory compositions of ketoprofen, amitriptyline and oxymetazoline, including, by way of example, the stable liquid pharmaceutical formulation described herein, may be prepared in accordance with the present invention with a concentration of active ingredients that is appropriate for direct injection into a localized site of inflammation, with the particular dosage being determined by a medical care provider. In one embodiment, the formulation of the present invention described above is not diluted or diluted no more than 10 parts diluent to one part of the formulation before administration to the patient. Suitable concentrations of active ingredients for direct injection in this concentrated form are in the following ranges: 40-750 µg/mL and more preferably 40-75 µg/mL ketoprofen, 12-240 µg/mL and more preferably 12-24 µg/mL amitriptyline HCl, and 12-240 µg/mL and more preferably 12-24 µg/mL oxymetazoline HCl.

In another embodiment, the formulation is diluted with a physiologic carrier, such as an irrigation vehicle, prior to injection into a localized site of injection. In this embodiment, the ketoprofen is present in the dilute injection solution at a concentration of no more than 500,000 nanomolar, preferably no more than 300,000 nanomolar, more preferably no more than 100,000 nanomolar and most preferably less than 50,000 nanomolar. The amitriptyline is suitably included in the dilute injection solution at a concentration of no more than 50,000 nanomolar, preferably no more than 30,000 nanomolar, more preferably no more than 25,000 nanomolar and most preferably less than 10,000 nanomolar. The oxymetazoline is suitably included in the dilute injection solution at a concentration of no more than 25,000 nanomolar, preferably no more than 20,000 nanomolar, more preferably no more than 15,000 nanomolar and most preferably less than 10,000 nanomolar. Still more preferably the diluted formulation for liquid injection includes 3.0-6.0 µg/mL and more preferably 4.0-5.0 µg/mL ketoprofen, 0.5-3.0 µg/mL and more preferably 1.0-2.0 µg/mL amitriptyline, and 0.5-3.0 µg/mL and more preferably 1.0-2.0 µg/mL oxymetazoline.

In another embodiment, the formulation of the present invention may be compounded into an oil or other biocompatible solvent, a suspension, a polymerizable or non-polymerizable gel, a paste or a salve for injection into a site of local inflammation. The formulation of the present invention may also be compounded into a carrier selected to enhance the delivery, uptake, stability or pharmacokinetics of the therapeutic agents, or with a sustained release delivery vehicle to form a depot upon local injection, such as microparticles, microspheres or nanoparticles composed of proteins, liposomes, carbohydrates, synthetic organic compounds, or inorganic compounds as well as polymerizable and non-polymerizable gels. The local injection formulations of the present invention may also be administered to a site of local inflammation by a continuous or intermittent pump.

Local injection of the formulation of the present invention in either concentrated form as described above, dilute form as described above, or any concentration there between, will be determined in accordance with the present invention by a medical practitioner provider depending on the nature of the injury. For example, in the case of treatment of an acute injury to a joint with an intact joint capsule, the dilute injection solution may be suitable. As an additional example, for an intratendinous injection or injection into tissues in which the residence time of the solution may be shorter, the concentrated injection solution may be preferred. The concentration and volume injected will be determined by the practitioner, based on characteristics of the anatomic site, such as whether the site is an enclosed space (e.g., a joint space), the volume of that space, the potential rate of diffusion, efflux or absorption of drug from the site of injection, etc., to ensure that an effective amount of the injected agents are retained at the local site to effectively inhibit inflammation at the site.

The volume of the formulation of the present invention, in concentrated or dilute form, injected into a site of local formulation will vary as required by the site and associated disorder to be treated and may suitably range between 1 mL and 30 mL, and more preferably between 5 mL and 20 mL. By way of non-limiting example, a volume of approximately 20 mL may be injected into a knee joint, approximately 20 mL may be injected into a subacromial space, approximately 10 mL may be injected into a shoulder joint, approximately 8-10 mL may be injected into a hip joint, approximately 5-10 mL may be injected into an ankle joint, approximately 3-5 mL may be injected into an elbow joint, approximately 1 mL may be injected into a wrist joint, approximately 10 mL may be injected into the greater trochanteric bursa and approximately 3-5 mL may be injected into the iliotibial band at the lateral femoral condyle.

The stable liquid pharmaceutical formulations can be diluted, e.g., into an irrigation vehicle for local delivery of the active pharmaceutical ingredients during arthroscopic surgery. For local delivery after dilution, the ketoprofen is at a concentration of no more than 500,000 nanomolar, preferably no more than 300,000 nanomolar, more preferably no more than 100,000 nanomolar and most preferably less than 50,000 nanomolar. For local delivery after dilution, the amitriptyline is suitably included at a concentration of no more than 50,000 nanomolar, preferably no more than 30,000 nanomolar, more preferably no more than 25,000 nanomolar and most preferably less than 10,000 nanomolar. For local delivery after dilution, the oxymetazoline is suitably included at a concentration of no more than 25,000 nanomolar, preferably no more than 20,000 nanomolar, more preferably no more than 15,000 nanomolar and most preferably less than 10,000 nanomolar. If the stable liquid pharmaceutical formulations are to be diluted into physiologic fluid that is used to irrigate a surgical site, those of skill will recognize that the concentration of active ingredients will be higher in the pharmaceutical formulation as compared to the delivered irrigation fluid. That is, the concentration of ketoprofen in the concentrated pharmaceutical formulation will be at least five, ten, one hundred, one hundred and fifty, five hundred or one thousand times greater than the diluted concentration of ketoprofen used to irrigate a surgical site. Similarly, the concentration of amitriptyline in the concentrated pharmaceutical formulation will be at least five, ten, one hundred, one hundred and fifty, five hundred or one thousand times greater than the diluted concentration or amitriptyline used to irrigate a surgical site. And the concentration of oxymetazoline in the concentrated pharmaceutical formulation will be at least five, ten, one hundred, one hundred and fifty, five hundred or one thousand times greater than the diluted concentration of oxymetazoline used to irrigate a surgical site. Table 1 provides exemplary concentrations of ketoprofen, amitriptyline, and oxymetazoline in a formulation prepared for later dilution into an irrigation vehicle.

The compositions of the present invention are typically formulated in an aqueous medium, but water is not required. PEG 400 or another polyol is included to aid in dissolution of the drugs, particularly ketoprofen. The PEG 400 or other polyol is present in concentrations between 1 and 100% (v/v). In some embodiments, the pharmaceutical formulation is diluted by a physician before administration to a patient, typically during an arthroscopic procedure. In an undiluted formulation, the PEG 400 concentration is typically between 2.0% and 99% (v/v). In preferred embodiments the PEG 400 concentration in the undiluted formulation is between 10% and 70% (v/v). In a more preferred embodiment, the PEG 400 concentration is between 15% and 50% (v/v). In another preferred embodiment, the PEG 400 concentration in the undiluted formulation is selected from 10%, 15%, 20%, 25%, 30%, 35%, 40%, 45%, or 50%. In a more preferred embodiment, the PEG 400 concentration in and undiluted formulation is 20% or about 20%.

Because of the involvement of divalent cations in catalyzing oxidation reactions, ethylenediamine tetraacetic acid (EDTA) can be included in the liquid pharmaceutical formulations disclosed herein. Typically the disodium salt, Na₂ EDTA, is used. The concentration of Na₂ EDTA is typically 0.01-0.5% (w/v). In one embodiment the Na₂ EDTA concentration is between 0.05-0.2%. In a preferred embodiment the Na₂ EDTA concentration is 0.05% or about 0.05%.

The liquid pharmaceutical formulations described herein suitably include citric acid as a buffering agent to maintain pH. Citric acid also has the ability to chelate divalent cations and can thus also prevent oxidation, thereby serving two functions as both a buffering agent and an antioxidant stabilizing agent. Citric acid is typically used in the form of a sodium salt. The concentration of Na citrate is typically 10-500 mM. In one embodiment, the concentration of Na citrate is about 50 mM.

For optimal stability of the active pharmaceutical ingredients, the pH of the stable liquid formulation is maintained at between 4.5-7.0, preferably between 5.0-6.2, more preferably between 5.2-5.8, and most preferably between 5.4-5.6. In another preferred embodiment, the pH is maintained at about 5.5.

Table 1 provides an exemplary formulation of the stable liquid pharmaceutical formulations disclosed herein. Sodium hydroxide and hydrochloric acid are added as necessary to adjust the pH to about 5.5. N₂ is laid over the final preparation to enhance stability. In a preferred embodiment, twenty milliliters of the formulation shown in Table 1 is diluted into 3,000 milliliters of arthroscopic irrigation solution and then administered to a patient during surgery.

**Table 1. Exemplary undiluted formulation in one liter final volume**

| **Component** | **Preferred Target Composition** | **Suitable Composition Range** |
|---|---|---|
| **Ketoprofen**, **USP** | 0.687 g/L | 0.550-0.824 g/L |
| **Amitriptyline HCl**, **USP** | 0.227 g/L | 0.182-0.272 g/L |
| **Oxymetazoline HCl, USP** | 0.215 g/L | 0.172-0.258 g/L |
| **PEG 400, NF** | 225.6 g/L | 180.5-270.7 g/L |
| **Citric acid monohydrate**, **USP** | 1.968 g/L | 1.589-2.362 g/L |
| **Na citrate dihydrate**, **USP** | 9.078 g/L | 7.262-10.894 g/L |
| **EDTA disodium**, **USP** | 0.500 g/L | 0.400-0.600 g/L |
| **Water for injection**, **USP** | 798.3 g/L | 639-958 g/L |
| **Sodium hydroxide, NF*** | --- | --- |
| **Hydrochloric acid, NF*** | --- | --- |
| **Nitrogen, NF*** | --- | --- |

| | | |
|---|---|---|
| *National formulary | | |

### Methods of use

The stable liquid formulations have application for a variety of operative/interventional procedures. The undiluted stable liquid formulations are stored until required by the user. In some embodiments, the formulations are then diluted into a physiologic irrigation vehicle and used to irrigate a surgical site during surgery, e.g., arthroscopic surgery or open surgery. The stable liquid formulations, either diluted or undiluted as appropriate, can also be injected directly into a site of localized inflammation. A site of localized inflammation can be, e.g., an acutely injured joint or other anatomic structure or a chronic site of inflammation, for example, an arthritic joint.

In one embodiment, the stable liquid pharmaceutical formulations described herein are diluted into a physiologic irrigation vehicle, which is then perioperatively applied during arthroscopic surgery of anatomic joints. Other applications include a variety of operative/interventional procedures, such as surgical, diagnostic and therapeutic techniques. As used herein, the term "perioperative" encompasses application intraprocedurally, pre- and intraprocedurally, intra- and postprocedurally, and pre-, intra- and postprocedurally. Preferably the irrigation solution is applied preprocedurally and/or postprocedurally as well as intraprocedurally. Physiologic irrigation fluids that are suitable vehicles for dilution of the formulation include normal saline, glycine, or lactated Ringer's solution, which are applied to the surgical site by techniques well known to those of ordinary skill in the art. The irrigation solution of the present invention is preferably applied to the wound or surgical site prior to the initiation of the procedure, preferably before tissue trauma, and continuously throughout the duration of the procedure, to preemptively block pain and inflammation. As used herein throughout, the term "irrigation" is intended to mean the flushing of a wound or anatomic structure with a stream of liquid. The term "application" is intended to encompass irrigation and other methods of locally introducing the formulations of the present invention. As used herein throughout, the term "continuously" is intended to also include situations in which there is repeated and frequent irrigation of wounds at a frequency sufficient to maintain a predetermined therapeutic local concentration of the applied agents, and applications in which there may be intermittent cessation of irrigation fluid flow necessitated by operating technique.

Once the stable liquid formulation is diluted for irrigation of a surgical site, the final, dilute concentration of the active pharmaceutical ingredients is as follows: ketoprofen preferably between 1,000-500,000 nM, more preferably between 5,000-100,000 nM; amitriptylin preferably between 100-50,000 nM, more preferably between 1,000-25,000 nM; and oxymetazoline preferably between 0.01-25,000 nM, more preferably between 0.05-15,000 nM. In a preferred embodiment, the dilute irrigation solution is prepared with the following concentration of active ingredients: 4.5 µg/mL ketoprofen, 1.50 µg/mL amitriptyline, and 1.42 µg/mL oxymetazoline. Those of skill will be able to determine an appropriate therapeutic dose of the ketoprofen, amitriptyline and oxymetazoline and will be able to calculate any necessary dilutions based on the composition of the stable liquid pharmaceutical formulation.

In addition to use during arthroscopic procedures, the disclosed formulations or pharmaceutical compositions, diluted into irrigation fluid as appropriate, may also be locally and perioperatively delivered during open surgical procedures on joints of the extremities, including but not limited total knee, hip, ankle, shoulder, elbow, wrist and interphalangeal joint replacements, the placement of implants into joints of the extremities, and for other surgical procedures on an extremity. As used herein, "extremity" refers to anatomic structures of the leg, including the hip, or of the arm, including the shoulder. Irrigation of open surgical sites at joints or extremities may be carried out in accordance with the invention by periodic direct irrigation with a bulb syringe or using other conventional techniques.

In preferred embodiments, the stable liquid pharmaceutical formulations disclosed herein are diluted in physiologic fluid and used to irrigate a surgical site during arthroscopic procedures. Arthroscopic techniques for which the present solution may be employed include, by way of non-limiting example, partial meniscectomies and ligament reconstructions in the knee, shoulder acromioplasties, rotator cuff debridements, elbow synovectomies, and wrist and ankle arthroscopies. The irrigation solution is continuously supplied intraoperatively to the joint at a flow rate sufficient to distend the joint capsule, to remove operative debris, and to enable unobstructed intra-articular visualization.

In another embodiment, compositions including a combination of ketoprofen, amitriptyline and oxymetazoline such as the stable liquid formulations described herein may be injected directly into a site of localized inflammation to inhibit, reduce or control inflammation at the site. The compositions of the present invention, in dilute or concentrated form, may be locally injected intra-articularly into an inflamed joint or joint at risk of inflammation, including a knee, hip, shoulder, elbow, ankle, wrist, temporomandibular, base of the thumb and small joints of the hands and feet and other articular joints. The compositions of the present invention may also be injected into a tendon or peritendinous area, ligament or periligamentous area, muscle or other soft tissue area that is inflamed or at risk of inflammation.

Locally inflamed sites that may be treated by direct injection in accordance with the present invention include joints, peritendinous areas and other anatomic structures that are affected by an acute condition, e.g., to treat intra-articular derangement, subluxation or dislocation of a joint, sprains, strains and for trigger-point injection. Local injection of the formulation and compositions of the present invention may also be used to treat subacute injuries, e.g., flexor tenosynovitis, or fasciitis such as plantar fasciitis. Local injection of the formulation and compositions of the present invention may also be used to treat chronic inflammatory diseases or conditions, e.g., arthritis, degenerative joint disease including osteoarthritis, rheumatoid arthritis and other arthitidies, synovitis, bursitis, tendonitis, carpal tunnel syndrome, gangliotic cysts, fibromyalgia, or repetitive or overuse injuries. Local injection of the formulation and compositions of the present invention may also be used prophylactically to inhibit anticipated injury such as injection into a joint of an athlete prior to participation of the athlete in a sporting event to avoid aggravation or recurrence of an old injury or condition.

Local injection of the formulation and compositions of the present invention is intended for the treatment of inflammatory conditions in the absence of a surgical procedure, such that the joint, peritendoinous area, muscles or other tissue into which the composition is injected are intact and the injected composition is retained in residence at the local site for a period of time sufficient for therapeutic effect.

Exemplary indications that may suitably be treated by local injection of the formulation and compositions of the present invention include, by way of non-limiting example, treatment to inhibit inflammation and pain following traumatic injury (e.g., joint derangement, meniscal tear); treatment to relieve postoperative inflammatory/pain disorders (e.g., arthrofibrosis, frozen shoulder); treatment for degenerative and overuse disorders (e.g., arthritis, subacromial impingement syndrome); intra-articular injection (e.g., arthrosis, intra-articular derangement, arthritis); trigger-point injection (e.g., myofascial pain syndrome); intrabursal injection (e.g., greater trochanteric bursa); treatment for friction syndromes (e.g., iliotibial band syndrome); tendon-sheath injection (e.g., flexor tenosynovitis, de Quervain's disease); intramuscular injection for muscle pain (e.g., musculofascial adhesions); and intratendinous injection for tendinitis (e.g., tendoachilles, patellar tendon).

Additionally, local injection of the formulation and compositions of the present invention may be administered in conjunction with other treatment modalities, such as in conjunction with musculoskeletal injections (e.g., corticosteroids, local anesthetics, platelet-rich plasma (PRP), interleukin-1 receptor antagonist (IL-1Ra) and anti-tumor necrosis factor alpha (anti-TNFα) antibodies, or for use in conjunction with the Graston Technique and fascial manipulation. When administered in conjunction with another treatment modality, the formulation and compositions of the present invention can be delivered prior to, together with or after the delivery of the other treatment to add to or enhance the effect, or mitigate one or more side effects, of the other treatment. Such administration of the formulation and compositions of the present invention could require a change in dosing to the other treatment.

A medical practitioner will determine the frequency of local injection of the formulations and compositions of the present invention. For example, a single injection may suffice immediately following an acute injury or for prophylactic purposes. For other acute, subacute and chronic conditions, an initial injection may be followed by a series of repeated injections on a daily, every other day, every several days or on a weekly basis, as needed. Injection can be completed under direct visualization or with the assistance of ultrasound or other imaging guidance.

The stable liquid formulations can be provided as part of a kit. Such a kit can include a vial of the stable liquid formulation, which can then be diluted into a carrier before use, e.g., in an irrigation fluid for a surgical procedure or for direct injection into a site of inflammation.

In another embodiment, the stable liquid formulations are sterile packaged in a syringe for direct injection into a site of localized inflammation, e.g., an acutely injured joint or a site of chronic inflammation. Because of the stability of the formulations disclosed herein, such syringes can be stored for a long period of time and kept on hand until needed by a physician or other health care provider.

In one embodiment, the stable liquid formulations for direct injection are prepared so that further dilution of active ingredients is not required before administration to a patient. The specific dose level for any particular patient will depend upon a variety of factors including the age, body weight, general health, the severity of the injury, and the identity of the injured joint. However, typically the dosage of active pharmaceutical ingredients will be less than the dosage for systemic administration and more than the dosage used in irrigation fluid for arthroscopic surgery, as disclosed herein. In a preferred embodiment, formulations for direct injection into an injured joint are prepared with the following concentration of active ingredients: 40-75 µg/mL ketoprofen, 12-24 µg/mL amitriptyline, and 12-24 µg/mL oxymetazoline. Those of skill can determine the appropriate therapeutic dose of the ketoprofen, amitriptyline and oxymetazoline for direct injection into an injured joint.

It must be noted that as used herein and in the appended claims, the singular forms "a", "and", and "the" include plural referents unless the context clearly dictates otherwise. Thus, for example, reference to "an excipient" includes a plurality of such excipients and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed. All citations are incorporated herein by reference.

### EXAMPLES

### Example 1: PEG 400 enhances solubility of the active pharmaceutical ingredients in liquid formulation.

Two liquid formulations of the active ingredients ketoprofen, amitriptyline HCl, and oxymetazoline HCl were prepared in the presence and absence of PEG 400. The composition of the liquid formulations, F1 and F2, are shown in Table 2. In both formulations, 50 mM sodium citrate buffer, pH 5.5 was used.

**Table 2. Solubility of liquid formulations.**

| **Formulation** | **Buffer** | **% PEG 400** (**v**/**v**) | **Measured solubility (Ketoprofen/Amitriptyline HCl/Oxymetazoline HCl)** | **Approximate saturation solubility (Ketoprofen/Amitriptyline HCl**/**Oxymetazoline HCl**) |
|---|---|---|---|---|
| F1 | 50 mM Na citrate, pH 5.5 | 0% | 0.687/0.227/0.215 (mg/mL) | 1.5 X measured solubility |
| F2 | 50 mM Na citrate, pH 5.5 | 20% | 1.370/0.452/0.427 (mg/mL) | 1.5 X measured solubility |

Addition of 20% PEG 400 (v/v) to the ketoprofen, amitriptyline HCl, and oxymetazoline HCl formulation resulted in measured solubility almost twice that of formulation without co-solvents. The results in Table 2 indicate that PEG 400 enhances the solubility of ketoprofen, amitriptyline HCl, and oxymetazoline HCl allowing preparation of concentrated formulations.

The solubility of ketoprofen, amitriptyline HCl, and oxymetazoline HCl in 20% PEG 400 (w/v) was compared solubility in the presence of 20% PEG 3350 (w/v). Three liquid formulations, F3, F4, and F5, were prepared with PEG concentrations shown in Table 3. Each of the three formulations included about 50 mM sodium citrate buffer, pH 5.5, ketoprofen (0.687 mg/mL), amitriptyline HCl (0.227 mg/mL), and oxymetazoline HCl, (0.227 mg/mL). The formulation was made on a one-liter pilot scale.

**Table 3. Dissolution and filtration times**

| **Formulation ID** | **Co-solvent added** | **Dissolution time for ketoprofen** | **Filtration time*** |
|---|---|---|---|
| F3 | None | ~ 4 hours /1-L scale | 23 sec. |
| F4 | 20% w/v PEG 400 | ~ 8 minutes/1-L scale | 56 sec. |
| F5 | 20% w/v PEG 3350 | ~ 4 hours /1-L scale | 183 sec. |

| | | | |
|---|---|---|---|
| Filtration time*: 100 mL of solution filtered through 0.22 µm 250-mL poly(vinylidine difluoride) (PVDF) filter under vacuum. | | | |

Ketoprofen is minimally soluble in water and took four hours to dissolve in either 50 mM Na citrate buffer pH 5.5 (F3), or 20% w/v of PEG 3350 (F5). PEG 3350 did enhance the solubility of ketoprofen, but did not reduce the time required for the dissolution of ketoprofen in the absence of any PEG. PEG 400 is a liquid at room temperature and ketoprofen dissolved in 100% PEG 400 within 8 minutes with stirring. After ketoprofen dissolution, 50 mM Na citrate buffer was added to bring the final PEG 400 concentration to 20% (w/v, F3).

The filtration time of the three solutions, F3, F4, and F5 was evaluated by filtering 100 milliliters of each solution through a 0.22 µm 250-mL PVDF filter under vacuum. Results are shown in Table 3. In the absence of any PEG, filtration of 100 milliliters of F3 took twenty-three seconds. Filtration of the 20% PEG 3350 (w/v) formulation (F5) took three minutes and three seconds under the same conditions. Filtration of the 20% PEG 400 (w/v) formulation (F4) took fifty-six seconds. Thus, addition of PEG 400 improved the dissolution of ketoprofen and did not significantly increase the filtration time of the formulation when compared to a buffer only formulation.

### Example 2: Citric acid buffer improves chemical stability of the three active pharmaceutical ingredients.

The stability of the active pharmaceutical ingredients was tested in acetate or citrate buffers. Gradient HPLC was used to quantify the three active pharmaceutical ingredients ketoprofen, amitriptyline HCl, and oxymetazoline HCl, and related substances in solution formulations after storage for up to eighty-four days at different temperatures. Aliquots of tested formulations were diluted into mobile phase to obtain a final concentration of about 0.0687 mg/mL to about 0.344 mg/mL ketoprofen, about 0.0227 mg/mL to about 0.114 mg/mL amitriptyline HCl, and about 0.0215 mg/mL to about 0.108 mg/mL oxymetazoline HCl. Chromatographic conditions for the related substances assay were as follows: (a) Detection wave length, UV 215 nm; (b) Column, Zorbax SB-C8, 5 µm, 4.6 x 250 mm; (c) Column temp, 30 ± 1 °C; (d) Sample temp, Ambient; (e) Flow rate, 1.2 mL/min; (f) Injection volume, 20 µL; (g) Run Time, 30 minutes. The gradient is shown in Figure 1A. Figure 1B shows the chromatogram of related substance assay for F7 stored at 4°C for about 4 months. Ketoprofen, amitriptyline HCl, and oxymetazoline HCl, have retention times of 12.572 minutes, 6.210 minutes, and 3.742 minutes respectively.

Two formulations, F6 and F7, were prepared with the buffers shown in Figure 2A. F6 included 50 mM Na acetate buffer, pH 5.5; F7 included 50 mM Na citrate buffer, pH 5.5. The other components of F6 and F7 were identical: Ketoprofen (0.687 mg/mL), Amitriptyline HCl (0.227 mg/mL), and Oxymetazoline HCl, and (0.227 mg/mL), and 20% PEG 400. Stability data is summarized in Figure 2B. When compared to 50 mM Na acetate, pH 5.5, the chemical stability of active ingredients, especially ketoprofen, was significantly improved when buffered with 50 mM sodium citrate, pH 5.5. Citric acid also has the ability to chelate divalent cations and can prevent oxidation, thus acting both as a buffering agent and an antioxidant.

### Example 3: Stability of the three active pharmaceutical ingredients is improved at pH 5.5.

The stability of the active pharmaceutical ingredients was tested in buffers with varying pH values. Ketoprofen, in particular, is more stable at higher pH. Data not shown. Two formulations, F8 and F9, were prepared with the buffers shown in Figure 3A. Both formulations included 50 mM Na citrate buffer. F8 pH was 6.5 and F9 pH was 5.5. The other components of F6 and F7 were identical: ketoprofen (0.687 mg/mL), amitriptyline HCl (0.227 mg/mL), and oxymetazoline HCl, and (0.227 mg/mL), and 20% PEG 400.

As above, gradient HPLC was used to quantify the three active pharmaceutical ingredients ketoprofen, amitriptyline HCl, and oxymetazoline HCl, and related substances in solution formulations after storage for eighty-four days at different temperatures. Results are shown in Figure 3B. The chemical stability of the active pharmaceutical ingredients, especially amitriptyline HCl and oxymetazoline HCl, was significantly improved when stored in a 50 mM Na citrate buffer at pH 5.5.

### Example 4: Chemical stability improves with N₂ overlay and protection from light during storage.

The stability of the active, pharmaceutical ingredients in liquid formulation was tested in the presence ofN₂ overlay and light protection. Two formulations, F10 and F11, were prepared and stored for up to 28 days under the conditions shown in Figure 4A, i.e., with or without N₂ overlay and light protection. Both formulations included 50 mM Na citrate buffer, pH 5.5, ketoprofen (0.687 mg/mL), amitriptyline HCl (0.227 mg/mL), and oxymetazoline HCl, and (0.227 mg/mL), and 20% PEG 400. F10 was prepared under protection from light and N₂ overlay post-compounding and pre-filling of formulation into vials, while F11 was not.

As above, gradient HPLC was used to quantify the three active pharmaceutical ingredients ketoprofen, amitriptyline HCl, and oxymetazoline HCl, and related substances in solution formulations after storage for twenty-eight days at different temperatures. Results are shown in Figure 4B. The chemical stability of the active pharmaceutical ingredients, especially oxymetazoline HCl and ketoprofen, was significantly improved when overlaid with N₂ after compounding and before vial filling, and when additionally protected from light during storage.

### Example 5: Addition of antioxidants improves chemical stability.

The effect of antioxidants on stability of the active pharmaceutical ingredients in liquid formulation was tested. Two formulations, F12 and F13, were prepared with the antioxidants shown in Figure 5A. F12 included 0.05% w/v Na₂ EDTA; no Na₂ EDTA was added to F13. Both formulations included 50 mM Na citrate buffer, pH 5.5, ketoprofen (0.687 mg/mL), amitriptyline HCl (0.227 mg/mL), and oxymetazoline HCl, and (0.227 mg/mL), and 20% PEG 400. In addition, both formulations were prepared under the same conditions, protected from light with N₂ overlay post-compounding and pre-filling of formulation into vials. Formulations containing Na bisulfite or Na metabisulfite were also tested.

As above, gradient HPLC was used to quantify the three active pharmaceutical ingredients ketoprofen, amitriptyline HCl, and oxymetazoline HCl, and related substances in solution formulations after storage for up to thirty days at different temperatures. Results are shown in Figure 5B. The chemical stability of especially amitriptyline HCl was improved in the presence of 0.05% w/v Na₂ EDTA. Disodium EDTA, a chelating agent, chelates divalent cations and prevents oxidation of active ingredients. As for the formulations containing Na bisulfite or Na metabisulfite, a large amitriptyline HCl-related substance peak was found in both samples after storage for thirty days. Data not shown. No further experiments were performed using Na bisulfite or Na metabisulfite antioxidant agents.

### Example 6: The liquid formulation of three active pharmaceutical ingredients is stable for at least two years.

The following formulation was selected for analysis of long-term storage at different temperatures: Na citrate, pH 5.5; 20% v/v PEG 400, 0.05% w/v Na₂ EDTA, ketoprofen (0.687 mg/mL), amitriptyline HCl (0.227 mg/mL), and oxymetazoline HCl (0.227 mg/mL). Ketoprofen was dissolved in 100% liquid PEG400 and was added to a solution of 50 mM Na citrate to bring the solution to final volume. Thus, the final concentration of Na citrate was about 50 mM. Three cGMP registration lots of the formulation, each in three hundred liter volume, were manufactured and stored at temperatures of 5°C, 25°C, 30°C, or 40°C for long-term stability evaluation. Aliquots were taken from each lot at intervals and analyzed for potency and presence of related substances. In addition, the lots were visually inspected for appearance and the pH was also monitored. No measurable changes in product color and appearance, pH of solution, or potency were detected over twenty-four months in any of the three registration lots stored at 5°C or 25°C. Data not shown.

Related substances (RS) were detected by HPLC. A summary of related substances data for one representative lot is shown in Figures 6A and 6B. After twenty-four months at 5°C, only one ketoprofen impurity was reproducibly above the reporting threshold of 0.08%. After twenty-four months at the 25°C three impurities, one from each of the three active pharmaceutical ingredients, were above the reporting threshold of 0.08%. The total related substances ranged from 0.21% to 0.35% after 24 months. Extrapolation of these results indicates that these formulations will be stable for at least three to four years, and possibly longer.

Oxymetazoline HCl in the liquid formulation remained stable for two years with a representative total RS% of 0.00% at 5°C and about 0.15% at 25°C. However, the total oxymetazoline HCl RS% increased significantly over two years when stored at 30°C and 40°C, indicating the degradation of oxymetazoline HCl is temperature-dependent.

At 25°C and 30°C, the percentage of amitriptyline HCl RS increased over the first three months, and then was stable for up to two years. At 40°C, the percentage of amitriptyline HCl RS increased during the first month and then stabilized. At 4°C, the amitriptyline HCl was present sporadically. These results indicate that amitriptyline HCl is stable in the tested formulation.

One ketoprofen impurity was slightly above the reporting threshold of 0.08%, and it did not increase over time at two accelerated temperature conditions, 30°C and 40°C. This result indicates that ketoprofen is also stable in the tested formulation under all four storage conditions.

### Example 7: The three active pharmaceutical ingredients are chemically compatible in the liquid formulation.

Chemical incompatibility between molecules results in molecular changes or rearrangements to form different chemical entities. Drugs may undergo a variety of chemical degradation pathways due to hydrolysis, oxidation (and reduction), and photodegradation. The compatibility of the active pharmaceutical ingredients with each other and with excipients was tested. The excipient formulation was Na citrate, pH 5.5; 20% v/v PEG 400, 0.05% w/v Na₂ EDTA. Active ingredients were added in the following concentrations: ketoprofen (0.687 mg/mL), amitriptyline HCl (0.227 mg/mL), and oxymetazoline HCl, and (0.227 mg/mL). The three active ingredients and excipient formulation, a vehicle (excipient) control formulation, and formulations of each individual drug substance in the vehicle control were evaluated for visual appearance (color and clarity), pH of solution, potency, and related substances. Each of the five formulations was prepared by simulating the final product manufacturing process and was filled into the container closure system proposed for commercial market. The test formulations were stored under both upright and inverted orientations at 5°C, 25°C, 30°C, and 40°C. Samples were analyzed immediately after production and at one-, two-, three-, and six-month time points.

Physical incompatibilities related to solubility changes or container interactions include precipitation, turbidity or haziness, changes in color or viscosity, and the formation of immiscible liquid layers. No such incompatibilities were observed in any of the tested formulations. Formulations in both upright and inverted orientations, at all temperatures, were clear and colorless solutions throughout the six-month study. Additionally, no change in pH was detected during the investigation.

Substances related to the active pharmaceutical ingredients were measured in each formulation. As temperatures increased, the number of related substances in each of the formulations increased. Five related substances (one from oxymetazoline HCl and four from ketoprofen) were observed in formulations held at 5°C, while eleven related substances (five from oxymetazoline HCl, two from amitriptyline HCl, and four from ketoprofen) were detected in formulations held at 25°C. All related substances found in the three-drug product were also found in the individual drug substance formulations. Thus, no new by-products or impurities were generated from interactions between the three active pharmaceutical ingredients or between the three active pharmaceutical ingredients and excipients in the formulation.

### Example 8: Additional liquid formulation stability studies.

Addition of Na₂ EDTA improved stability of the active ingredients, especially amitriptyline HCl. Formulations without Na₂ EDTA were also tested for long term stability. An additional long term study was performed to determine the stability of a candidate formulation without sodium Na₂ EDTA at one-liter scale as compared to a similar formulation with Na₂ EDTA. In the previous formulation screening studies, glass volumetric flasks were used for the preparation of all prototype formulations. Excess volume of buffer solutions, such as Na acetate and Na citrate buffers, were made prior to mixing with the ketoprofen PEG 400 solution, and were used to QS to the target formulation volume. Therefore, the exact quantity of citric acid monohydrate and Na citrate dihydrate in the final formulation solution was not known.

To produce a drug product using good manufacturing practice (GMP), it is necessary to know the precise amount of each drug product component. Therefore, solid citric acid monohydrate and sodium citrate dihydrate were added to the aqueous formulations. At the one-liter pilot formulation scale, the batch quantity of the three active pharmaceutical ingredients, ketoprofen, amitriptyline HCl, and oxymetazoline HCl, were 687.0 mg/L, 226.5 mg/L, and 214.5 mg/L. Twenty percent PEG 400 was included. Ketoprofen was dissolved in 100% PEG 400. The density of PEG 400 is 1.128 g/mL at 25°C. Therefore, 20% v/v of PEG 400 at 1-L scale (200 mL) is 225.60 g/L. The batch quantities of citric acid monohydrate and Na citrate dihydrate were calculated to be 1.969 g/L and 9.086 g/L respectively. The total weight of one liter of formulation lacking Na₂ EDTA was determined to be 1036.09 grams with a density of approximately 1.036 g/mL. The batch quantity of the water for injection was calculated to be 799.00 g/L. For the formulation with Na₂ EDTA, 0.500 g/L of Na₂ EDTA was added. Because the batch quantity of Na₂ EDTA is minimal, the density of formulation without Na₂ EDTA is assumed to be the same as that of formulation with Na₂ EDTA, 1.036 g/mL.

Production of the formulations was carried out as follows. First, 687 mg of ketoprofen USP was placed in a one-liter stainless steel container. With mixing, 225.6 g of PEG 400 was added to the ketoprofen in the vessel, and mixed for a minimum of five minutes. If not dissolved, mixing was continued until dissolution was complete. The amount of PEG 400 added, and mixing time needed to dissolve the ketoprofen was recorded. With mixing, the batch quantity 799.0 g of water for injection (WFI), was added to the ketoprofen/PEG 400 solution in the vessel and mixed for a minimum of five minutes, or until a clear solution was obtained.

With mixing, the batch quantity 1.969 g of citric acid monohydrate, USP was added to the solution in the vessel, and mixed for a minimum of five minutes or until dissolved. With mixing, the batch quantity 9.086 g of Na citrate dihydrate, USP was added to the solution in the vessel, and mixed for a minimum of five minutes or until dissolved. With mixing, the batch quantity 0.500 g of Na₂ EDTA, USP was added to the solution in the vessel, and mixed for a minimum of 5 minutes or until dissolved. Disodium EDTA was added to only one formulation.

With mixing, the batch quantity 226.5 mg of amitriptyline HCl, USP was added to the solution in the vessel, and mixed for a minimum of five minutes or until dissolved. With mixing, the batch quantity 214.5 mg of oxymetazoline HCl, USP was added to the solution in the vessel, and mixed for a minimum of five minutes or until dissolved. The pH of the final formulation was measured. Fifty milliliters of the solution was filtered through a 0.22 µm Durapore filter into a clean receiving vessel as a conditioning step, and the filter was discarded. The remaining solution was filtered through the preconditioned 0.22 µm Durapore filter into the receiving vessel, and the filtration time was recorded.

Approximately 20 mL of the solution from each formulation were dispensed into one of two labeled vials. The vial headspace was purged with N₂ (N₂ overlay). A stopper was placed into each filled vial, and sealed with aluminum seals. The vials were labeled, and placed in the 5°C stability chambers, and were used as the t = 0 hold stability samples.

For N₂ overlay post-compounding and pre-filling: The 1-liter vessel headspace was purged with N₂ for about five minutes, and the top of the vessel was sealed with parafilm. For N₂ sparge post-compounding and pre-filling: The 1-liter solution was sparged with N₂ for 30 minutes while stirring, and the top of the vessel was sealed with parafilm.

At eight hours, approximately 20 mL of the solution were withdrawn from the vessel into each of two labeled vials, and the vial headspace was purged with N₂ (N₂ overlay). A stopper was placed into each filled vial, and sealed with aluminum seals. The vials were labeled, and placed in 5°C stability chambers prior to HPLC analysis.

After twenty-four hours, approximately 20 mL of the solution was withdrawn from the vessel into each of two labeled vials, and the vial headspace was purged with N₂ (N₂ overlay). A stopper was placed into each filled vial, and sealed with aluminum seals. The vials were labeled, and placed in the 5°C stability chambers prior to HPLC analysis.

About 20 mL of the formulation was dispensed into the specified vials, and the vial headspace was purged with N₂ for fifteen seconds (N₂ overlay). A stopper was placed into each filled vial, and sealed with aluminum seals. The vials were labeled, and placed in the following stability chambers at 5°C, 25°C, 30°C, and 40°C. At each time point, one sample per formulation per storage condition was pulled and submitted for visual analysis, pH analysis, potency assay of active ingredients (i.e., ketoprofen, amitriptyline HCl and oxymetazoline HCl), and related substances. The 1-liter vessel headspace of the remaining solution was purged with N₂ (N₂ overlay) for about five minutes, and the top of the vessel was sealed with parafilm.

At 32 hours, approximately 20 mL of the solution were withdrawn from the vessel into each of two labeled vials, and the vial headspace was purged with N₂ (N₂ overlay). A stopper was placed into each filled vial, and sealed with aluminum seals. The vials were labeled, and placed in the 5°C stability chambers prior to HPLC analysis.

At 48 hours, approximately 20 mL of the solution were withdrawn from the vessel into each of two labeled vials, and the vial headspace was purged with N₂ (N₂ overlay). A stopper was placed into each filled vial, and sealed with aluminum seals. The vials were labeled, and placed in the 5°C stability chambers prior to HPLC analysis.

### Results

Ketoprofen was easily dissolved into the PEG 400 solutions by briefly stirring for 6-10 minutes. The measured pH values of the no-Na₂ EDTA formulations N₂ overlay and N₂ sparge, were about 5.76, due to the basic nature of PEG 400. The measured pH values of the Na₂ EDTA-containing formulations, N₂ overlay and N₂ sparge, were around 5.73, only slightly lower than 5.76 of no-Na₂ EDTA formulations, because of the addition of acidic Na₂ EDTA. All four formulations were clear solutions after compounding, and remained clear during storage by visual analysis.

The compounded bulk stability of the formulations at room temperature was determined at time points of 0, 8, 24, 32 and 48 hours. As assayed by potency and absence of related substances, all four formulations were stable over the 48- hour hold period.

After the 48- hour compounded bulk hold study, all four lots of material were placed on a stability program and were tested for potency and related substances. After six months, the potency data resulting from these tests did not show any significant changes.

Oxymetazoline HCl related substances were very low over six months, e.g., less than 1%. At 60°C all the formulations showed at least some minimal oxymetazoline HCl degradation at 60°C.

Amitriptyline HCl degraded more than oxymetazoline HCl over six months. The formulations with Na₂ EDTA had less amitriptyline HCl degradation than the formulations without Na₂ EDTA. In all samples, amitriptyline HCl related substances were present at less than 5%. Amitriptyline HCl degradation did not appear to be affected by N₂ sparging during sample compounding.

Ketoprofen degradation was minimal after six months, even at the accelerated stability temperatures. Related substances were present at less than 1.5%.

As shown in Figure 7, total related substances after six months were minimal in all formulations, i.e., 3% or less. N₂ sparging of the bulk formulation during compounding had no apparent effect on final product stability.

## Claims

1. A stable liquid pharmaceutical formulation comprising a solution of 550 µM to 1 M ketoprofen, 55 µM to 1 M amitriptyline, 350 µM to 1 M oxymetazoline, 1-100% (v/v) polyol, and 10-500 mM Na citrate, pH 4.5-7.0.

2. The pharmaceutical formulation of claim 1, wherein the polyol concentration is 15-25% (v/v).

3. The pharmaceutical formulation of claim 1, wherein the polyol is PEG 400.

4. The pharmaceutical formulation of claim 1, wherein the formulation is an aqueous formulation comprising 75-85% (v/v) water.

5. The pharmaceutical formulation of claim 1, wherein the pH is between 5.2 and 5.8.

6. The pharmaceutical formulation of claim 1, wherein the pH is between 5.4 and 5.6.

7. The pharmaceutical formulation of claim 1, wherein the ketoprofen, amitriptyline, oxymetazoline are stable for at least six months, for example, at least twelve months when stored at a temperature of between 2°C and 30°C.

8. The pharmaceutical formulation of claim 1, wherein the ketoprofen, amitriptyline, oxymetazoline are stable for at least twenty-four months when stored at a temperature of between 2°C and 30°C.

9. The pharmaceutical formulation of claim 1, further comprising 0.01-0.5% (w/v) Na2 EDTA.

10. The pharmaceutical formulation of claim 1, wherein the concentration of Na2 EDTA is 0.05% (w/v).

11. The pharmaceutical formulation of claim 1, wherein the Na Citrate concentration is about 50 mM.

12. A liquid pharmaceutical formulation comprising 0.1-1.0 g/L ketoprofen, 0.05-0.5 g/L amitriptyline, 0.05-0.5 g/L oxymetazoline, about 0.002-1.0% (w/v) Na₂EDTA, and 10-500 mM Na citrate, in a medium comprising 10-70% (v/v) PEG 400 and 30-90% v/v water, with a pH of 5.0-6.2, wherein the ketoprofen, amitriptyline, and oxymetazoline are soluble, and wherein the ketoprofen, amitriptyline, oxymetazoline are stable for at least six months when stored at a temperature of between 2°C and 30°C.

## Patentansprüche

1. Stabile flüssige pharmazeutische Formulierung, umfassend eine Lösung aus 550 µM bis 1 M Ketoprofen, 55 µM bis 1 M Amitriptylin, 350 µM bis 1 M Oxymetazolin, 1-100 Vol.-% Polyol und 10-500 mM Na-Citrat, pH-Wert 4,5-7,0.

2. Pharmazeutische Formulierung nach Anspruch 1, wobei die Polyolkonzentration 15-25 Vol.-% beträgt.

3. Pharmazeutische Formulierung nach Anspruch 1, wobei das Polyol PEG 400 ist.

4. Pharmazeutische Formulierung nach Anspruch 1, wobei die Formulierung eine wässrige Formulierung, umfassend 75-85 Vol.-% Wasser, ist.

5. Pharmazeutische Formulierung nach Anspruch 1, wobei der pH-Wert zwischen 5,2 und 5,8 beträgt.

6. Pharmazeutische Formulierung nach Anspruch 1, wobei der pH-Wert zwischen 5,4 und 5,6 beträgt.

7. Pharmazeutische Formulierung nach Anspruch 1, wobei das Ketoprofen, das Amitriptylin und das Oxymetazolin wenigstens sechs Monate lang stabil sind, beispielsweise wenigstens 12 Monate lang, wenn bei einer Temperatur zwischen 2 °C und 30 °C aufbewahrt.

8. Pharmazeutische Formulierung nach Anspruch 1, wobei das Ketoprofen, das Amitriptylin und das Oxymetazolin wenigstens vierundzwanzig Monate lang stabil sind, wenn bei einer Temperatur zwischen 2 °C und 30 °C aufbewahrt.

9. Pharmazeutische Formulierung nach Anspruch 1, ferner umfassend 0,01-0,5 % (w/v) Na₂-EDTA.

10. Pharmazeutische Formulierung nach Anspruch 1, wobei die Konzentration von Na₂-EDTA 0,05 % (w/v)beträgt.

11. Pharmazeutische Formulierung nach Anspruch 1, wobei die Na-Citrat-Konzentration etwa 50 mM beträgt.

12. Flüssige pharmazeutische Formulierung, umfassend 0,1-1,0 g/l Ketoprofen, 0,05-0,5 g/l Amitriptylin, 0,05-0,5 g/l Oxymetazolin, etwa 0,002-1,0 % (w/v) Na₂-EDTA und 10-500 mM Na-Citrat, in einem Medium, das 10-70 Vol.-% PEG 400 und 30-90 Vol.-% Wasser umfasst, mit einem pH-Wert von 5,0-6,2, wobei das Ketoprofen, das Amitriptylin und das Oxymetazolin löslich sind und wobei das Ketoprofen, das Amitriptylin, und das Oxymetazolin wenigstens sechs Monate lang stabil sind, wenn bei einer Temperatur zwischen 2 °C und 30 °C aufbewahrt.

## Revendications

1. Formulation pharmaceutique liquide stable comprenant une solution de 550 µM à 1 M de kétoprofène, 55 µM à 1 M d'amitriptyline, 350 µM à 1 M d'oxymétazoline, 1-100 % (v/v) de polyol et 10-500 mM de citrate de Na, à un pH de 4,5-7,0.

2. Formulation pharmaceutique selon la revendication 1, dans laquelle la concentration en polyol est de 15-25 % (v/v).

3. Formulation pharmaceutique selon la revendication 1, dans laquelle le polyol est le PEG 400.

4. Formulation pharmaceutique selon la revendication 1, dans laquelle la formulation est une formulation aqueuse comprenant 75-85 % (v/v) d'eau.

5. Formulation pharmaceutique selon la revendication 1, dans laquelle le pH se situe entre 5,2 et 5,8.

6. Formulation pharmaceutique selon la revendication 1, dans laquelle le pH se situe entre 5,4 et 5,6.

7. Formulation pharmaceutique selon la revendication 1, dans laquelle le kétoprofène, l'amitriptyline et l'oxymétazoline sont stables pendant au moins six mois, par exemple, au moins douze mois lors d'un entreposage à une température entre 2 °C et 30 °C.

8. Formulation pharmaceutique selon la revendication 1, dans laquelle le kétoprofène, l'amitriptyline et l'oxymétazoline sont stables pendant au moins vingt-quatre mois lors d'un entreposage à une température entre 2 °C et 30 °C.

9. Formulation pharmaceutique selon la revendication 1, comprenant en outre 0,01-0,5 % (p/v) de Na₂ EDTA.

10. Formulation pharmaceutique selon la revendication 1, dans laquelle la concentration en Na₂ EDTA est de 0,05 % (p/v).

11. Formulation pharmaceutique selon la revendication 1, dans laquelle la concentration en citrate de Na est d'environ 50 mM.

12. Formulation pharmaceutique liquide comprenant 0,1-1,0 g/l de kétoprofène, 0,05-0,5 g/l d'amitriptyline, 0,05-0,5 g/l d'oxymétazoline, environ 0,002-1,0 % (p/v) de Na₂ EDTA et 10-500 mM de citrate de Na, dans un milieu comprenant 10-70 % (v/v) de PEG 400 et 30-90 % v/v d'eau, avec un pH de 5,0-6,2, dans laquelle le kétoprofène, l'amitriptyline et l'oxymétazoline sont solubles et dans laquelle le kétoprofène, l'amitriptyline et l'oxymétazoline sont stables pendant au moins six mois lors d'un entreposage à une température entre 2 °C et 30 °C.
